**Europäisches Patentamt**

**European Patent Office**

**Office européen des brevets**

(19)

(11) Veröffentlichungsnummer : **0 394 984 B1**

(12)
# EUROPÄISCHE PATENTSCHRIFT

(45) Veröffentlichungstag der Patentschrift :
09.06.93 Patentblatt 93/23

(51) Int. Cl.⁵ : **C07C 41/34,** C07C 43/11,
C07C 43/13, C08G 65/30

(21) Anmeldenummer : **90107814.7**

(22) Anmeldetag : **25.04.90**

(54) **Verfahren zur Reinigung von Alkylenoxid-Addukten.**

(30) Priorität : 27.04.89 DE 3913937

(43) Veröffentlichungstag der Anmeldung :
31.10.90 Patentblatt 90/44

(45) Bekanntmachung des Hinweises auf die
Patenterteilung :
09.06.93 Patentblatt 93/23

(84) Benannte Vertragsstaaten :
AT BE CH DE ES FR GB IT LI NL SE

(56) Entgegenhaltungen :
JP-A-54 109 907
US-A- 3 000 963
US-A- 4 306 943

(73) Patentinhaber : HOECHST
AKTIENGESELLSCHAFT
Postfach 80 03 20
W-6230 Frankfurt am Main 80 (DE)

(72) Erfinder : Wimmer, Ignaz, Dr.
Burger Strasse 26
W-8261 Winhöring (DE)
Erfinder : Billenstein, Siegfried, Dr.
Samerbergweg 8
W-8269 Burgkirchen (DE)

EP 0 394 984 B1

## Beschreibung

Die Erfindung betrifft ein Verfahren zur Reinigung eines Alkylenoxid-Adduktes, das unter Verwendung von halogenhaltigen Metallkatalysatoren hergestellt worden ist und Halogen enthaltende Verbindungen sowie Metalle als Verunreinigung enthält.

Es ist schon seit langem bekannt, daß die Alkoxylierung von ein- oder mehrwertigen Alkoholen unter Verwendung von halogenhaltigen Metallkatalysatoren, vorzugsweise aus der Gruppe der Antimon- und Zinnhalogenide (die in der Regel in Form von $SbCl_5$ und $SnCl_4$ eingesetzt werden) eine wesentlich engere Molmassenverteilung des Oxalkylates liefert, als unter Verwendung von basischen Katalysatoren. Diesem beträchtlichen Vorteil steht jedoch der Nachteil entgegen, daß das Oxalkylat die eingesetzten Katalysatormetalle, wie Antimon und/oder Zinn, sowie anorganisch und/oder organisch gebundenes Halogen als besonders unerwünschte Verunreinigungen enthält.

Es sind auch schon mehrere Verfahren bekannt, die sich mit der Reinigung der in Rede stehenden Oxalkylate beschäftigen. So wird in der japanischen Offenlegungsschrift 54-109907 (vergleiche Derwent-Referat CPI-Nr. 72865B/40) ein Verfahren zum Reinigen von mit Zinntetrachlorid als Katalysator hergestellten Alkylenoxid-Addukten beschrieben, bei dem das Addukt (Rohoxalkylat) mit einem Phosphat oder mit Phosphorsäure in einer Menge von 0,1 bis 5 Gew.-%, bezogen auf das Gewicht des Adduktes, behandelt wird, wobei anwesendes Zinntetrachlorid in das abfiltrierbare Zinnphosphat übergeführt wird. Mit diesem Verfahren wird zwar eine relativ gute Reinigung des Rohoxalkylates bezuglich Zinn erreicht, nicht aber auch bezüglich der genannten Halogenverbindungen, die ebenfalls besonders störende Verunreinigungen darstellen.

Wie aus der genannten japanischen Offenlegungsschrift hervorgeht, ist auch schon versucht worden, die in Rede stehende Reinigung durch Behandeln des Rohoxalkylates mit Alkalimetallverbindungen durchzuführen, wobei jedoch die angestrebte Reinheit nicht erreicht wurde und das behandelte Oxalkylat sogar Trübheit, Fluoreszenz und/oder Fällungserscheinungen aufwies. Diese negativen Ergebnisse werden in zwei Vergleichsversuchen bestätigt, in denen das zu reinigende Alkylenoxid-Addukt mit einer bestimmten Menge an Kaliumhydroxid und an Natriummethylat jeweils 30 Minuten lang bei 120 °C gerührt worden ist.

Es wurde nun ein Verfahren zur Behandlung der in Rede stehenden Alkylenoxid-Addukte (Rohoxalkylate) gefunden, mit dem eine praktisch vollständige Reinigung sowohl hinsichtlich Metallen, wie Antimon und/oder Zinn, als auch hinsichtlich Halogen enthaltenden Verbindungen erreicht werden kann. Mit dem erfindungsgemäßen Verfahren wird insbesondere auch das organisch gebundene Halogen erfaßt und entfernt. Das erfindungsgemäß erhaltene Produkt besitzt neben der genannten hohen Reinheit auch ein klares Aussehen und ist geruchlos.

Das erfindungsgemäße Verfahren zur Reinigung eines Alkylenoxid-Adduktes, das unter Verwendung von halogenhaltigen Metallkatalysatoren hergestellt worden ist und Halogen enthaltende Verbindungen sowie Metalle als Verunreinigung enthält, ist dadurch gekennzeichnet, daß man

a) das zu reinigende Alkylenoxid-Addukt mit Alkalimetallverbindungen aus der Gruppe der Oxide, Hydroxide und Alkoholate in einer mindestens 1,05fachen stöchiometrischen Menge, bezogen auf die im Addukt vorliegende Halogenmenge, bei erhöhter Temperatur in Kontakt bringt,

b) das alkalische Produkt vom Schritt a) mit einer Säure auf einen pH-Wert von höchstens 7 bringt, mit der Maßgabe, daß im nun vorliegenden Produkt eine Wassermenge von mindestens 0,1 Gew.-%, bezogen auf das Gewicht des Produktes, anwesend ist,

c) aus dem nach den Schritten a) und b) erhaltenen Produkt das Wasser und andere Lösungsmittel für die in den Schritten a) und b) gebildeten und im Alkylenoxid-Addukt unlöslichen Salze entfernt, und man

d) aus dem im Schritt c) erhaltenen Produkt, das im wesentlichen aus Alkylenoxid-Addukt und den genannten Salzen besteht, durch Abtrennung dieser Salze das angestrebte reine Alkylenoxid-Addukt gewinnt.

Mit dem erfindungsgemäßen Verfahren wird ein Alkylenoxid-Addukt erhalten, das praktisch frei ist von den Metallen, wie Antimon und Zinn, sowie von anorganisch und organisch gebundenem Halogen (organisch gebundenes Halogen, das den Großteil am Gesamthalogengehalt darstellt, liegt in organischen Halogenverbindungen vor, die im wesentlichen durch die halogenierende Wirkung der halogenhaltigen Katalysatoren auf die organischen Verbindungen, wie Alkohol, Polyol, Alkylenoxid und dergleichen, entstehen). Das erfindungsgemäß erhaltene Produkt ist auch geruchlos und von klarem Aussehen. Diese Ergebnisse sind um so überraschender, als in der genannten japanischen Offenlegungsschrift nicht nur von der Verwendung von Alkalimetallverbindungen allein als Reinigungsmittel wiederholt abgeraten wird, sondern auch von der Verwendung einer Kombination von Alkalimetallverbindung (Alkalimetallhydroxid) und Säure (Phosphorsäure), die zu einem denaturierten Addukt führen soll.

Im Schritt a) des erfindungsgemäßen Verfahrens werden vorzugsweise Alkalimetallhydroxide und Alkalimetallalkoholate der $C_1$ bis $C_4$-Alkanole, insbesondere von Methanol und Ethanol, eingesetzt. Die Alkalimetalle sind aus Zweckmäßigkeitsgründen in der Regel Kalium und Natrium. Die Alkalimetallverbindung wird vorzugs-

2

weise in einer 1,3- bis 5fachen stöchiometrischen Menge, bezogen auf die Menge an Halogen in dem zu reinigenden Addukt, eingesetzt (bei Halogen handelt es sich klarerweise in der Regel um Chlor). Die Verwendung einer noch größeren Menge an Alkalimetallverbindung ist ohne weiteres möglich, im allgemeinen aber nicht zweckmäßig. Die Alkalimetallverbindung wird ferner vorzugsweise in Form einer Lösung eingesetzt, wobei Wasser, ein $C_1$ bis $C_4$-Alkanol oder eine Mischung aus Wasser und einem $C_1$ bis $C_4$-Alkanol die bevorzugten Lösungsmittel sind. Als Alkanol ist Methanol oder Ethanol besonders bevorzugt. Im Falle der Verwendung von Wasser und Alkohol als Lösungsmittel kann das Gewichtsverhältnis der beiden in weiten Grenzen variieren; es kann zum Beispiel im Bereich von 1 : 99 bis 99 : 1 liegen, es liegt im allgemeinen im Bereich von 1 : 70 bis 70 : 1. Die Konzentration der Lösungen an Alkalimetallverbindung ist ebenfalls nicht kritisch. Sie liegt aus Zweckmäßigkeitsgründen bei 1 bis 70 Gew.-%, vorzugsweise bei 5 bis 50 Gew.-%, Gewichtsprozente bezogen auf das Gewicht der wäßrigen, alkoholischen oder wäßrig/alkoholischen Lösung. Nach einer bevorzugten Ausführungsform des Schrittes a) wird die Behandlung des Rohoxalkylates mit der Alkalimetallverbindung in der Weise durchgeführt, daß die Mischung aus Rohoxalkylat und Alkalimetallverbindung bei erhöhter Temperatur, vorzugsweise bei einer Temperatur von 80 bis 180 °C, insbesondere bei 100 bis 150 °C, 0,5 bis 5 Stunden lang gerührt wird, vorzugsweise unter Aufrechterhaltung einer Inertgasatmosphäre, zum Beispiel Stickstoffatmosphäre, zwecks Ausschluß von Sauerstoff. Mit dem Schritt a) des erfindungsgemäßen Verfahrens wird beispielsweise Antimon in Antimonat und Zinn in Stannat und alles anorganische und organisch gebundene Halogen in Alkalimetallhalogenid (das ist in der Regel Natrium- oder Kaliumchlorid) umgewandelt.

Im Schritt b) des erfindungsgemäßen Verfahrens wird die nach Ausführung des Schrittes a) erhaltene Rohoxalkylat-Mischung mit einer Säure versetzt, vorzugsweise unter Rühren, bis ein pH-Wert von höchstens 7 vorliegt, vorzugsweise von 3 bis 7, insbesondere von 4 bis 6,5. Zum Ansäuern können anorganische oder organische Säuren sowie deren saure Salze, wie Alkali-, Amin- oder Ammoniumsalze, eingesetzt werden. Bevorzugte Säuren sind jene aus der Gruppe der Phosphorsäuren, Schwefelsäuren, Oxalsäure und Citronensäure, wobei die Phosphorsäuren, Schwefelsäuren und Oxalsäure bevorzugt sind. Aus der Gruppe der Phosphorsäuren werden vorzugsweise die Phosphorsäure ($H_3PO_4$), die phosphorige Säure ($H_3PO_3$) und die unterphosphorige Säure ($H_3PO_2$) eingesetzt und aus der Gruppe der Schwefelsäuren die Schwefelsäure ($H_2SO_4$) und die schwefelige Säure ($H_2SO_3$). Die Säure kann als solche oder in verdünnter Form, vorzugsweise als 20- bis 90gew.%ige wäßrige Lösung, eingesetzt werden. Das in Rede stehende Ansäuern kann bei Raumtemperatur oder bei erhöhter Temperatur durchgeführt werden. Es wird vorzugsweise bei einer Temperatur von 20 bis 150 °C und insbesondere bei einer Temperatur von 50 bis 130 °C durchgeführt. Mit der beschriebenen Einstellung des pH-Wertes wird erreicht, daß beispielsweise Antimonat und Stannat und das überschüssige Alkalimetallhydroxid und/oder -alkoholat in das der eingesetzten Säure entsprechende Salz umgewandelt wird.

Die nach der Einstellung des pH-Wertes vorliegende Mischung soll einen Wassergehalt von mindestens 0,1 Gew.-% besitzen, vorzugsweise von 0,5 bis 10 Gew.-%, Gewichtsprozente bezogen auf das Gewicht der Mischung. Dieser Wassergehalt wird beispielsweise dadurch erreicht, daß im Schritt a) eine wäßrige oder wäßrig/alkoholische Lösung eingesetzt wird und/oder daß das Ansäuern im Schritt b) mit einer wäßrigen Säurelösung durchgeführt wird. Wenn weder im Schritt a) noch beim genannten Ansäuern im Schritt b) Wasser in das Rohoxalkylat eingebracht wird (und deswegen praktisch kein Wasser anwesend ist), wird man die geforderte Wassermenge nun zusetzen, das heißt vor Durchführung des Schrittes c).

Im Schritt c) des erfindungsgemäßen Verfahrens wird die Produktmischung, wie sie nach Durchführung des Schrittes b) vorliegt, von den Lösungsmitteln (in denen die anwesenden Salze teils oder ganz gelöst sind) befreit. Bei diesen Lösungsmitteln handelt es sich im wesentlichen um Wasser und Alkohol, insbesondere um Wasser. Die Entfernung des Wassers und gegebenenfalls des Alkohols wird vorzugsweise destillativ durchgeführt. Die destillative Lösungsmittelentfernung kann unter Erhitzen der Mischung, vorzugsweise auf 100 bis 130 °C, und/oder mit Hilfe von Vakuum durchgeführt werden, wobei zur Beschleunigung ein Schleppgas (Stickstoff) eingesetzt werden kann. Mit einem hohen Vakuum kann die Lösungsmittelentfernung auch bei Raumtemperatur erreicht werden. In der Regel werden mit der Entfernung des Wassers gleichzeitig auch andere flüchtige Anteile, beispielsweise die obengenannten Alkohole, aus der Mischung ausgetragen.

Nach der Entfernung von Wasser und Alkohol aus der Mischung liegen alle im Alkylenoxid-Addukt unlöslichen Salze in einer derart kristallinen Form vor, daß sie vom Alkylenoxid-Addukt leicht und vollständig abgetrennt werden können. Die Forderung der Gegenwart von Wasser in der Produktmischung vor ihrer Behandlung im Schritt c) resultiert aus der Feststellung, daß die in Rede stehenden Salze dann relativ grob-kristallin und damit in einer leicht und vollständig abtrennbaren Form erhalten werden, wenn sie wenigstens teilweise zur Auskristallisation aus Wasser gebracht werden. Die Wassermenge in der Produktmischung braucht nicht so groß zu sein, daß die gesamte Menge der anwesenden Salze darin gelöst ist; es kann andererseits viel mehr Wasser als die zur Lösung der gesamten Salze erforderliche Menge eingesetzt werden. Die bevorzugte Wassermenge liegt bei etwa 0,5 bis 10 Gew.-%, Gewichtsprozente bezogen auf das Gewicht der Produktmischung. Eine größere Wassermenge als die angegebenen 10 Gew.-% ist also ohne weiteres möglich, sie ist aber unzweckmäßig, weil dann im Schritt c) mehr Wasser zu entfernen ist. In beiden Fällen, das heißt sowohl im Falle von relativ wenig Wasser als auch im Falle von relativ viel Wasser, wird im Schritt c) ein grob-kristallines Salz erreicht.

Im Schritt d) des erfindungsgemäßen Verfahrens erfolgt die Abtrennung der im Schritt c) auskristallisierten Verbindungen. Diese Abtrennung und Gewinnung des angestrebten hoch reinen Alkylenoxid-Adduktes wird vorzugsweise durch Filtrieren oder Zentrifugieren der Produktmischung durchgeführt. Im Falle, daß die Produktmischung bei Raumtemperatur fest oder hoch viskos ist, wird die genannte Abtrennung klarerweise bei einer solchen Temperatur durchgeführt, bei der das Alkylenoxid-Addukt die gewünschte Fließbarkeit aufweist.

Die Art und Zusammensetzung des Alkylenoxid-Adduktes, das nach dem erfindungsgemäßen Verfahren behandelt wird, ist nicht kritisch. Das erfindungsgemäße Reinigungsverfahren kann auf verschiedene Rohoxalkylate angewandt werden. In der Regel wird es sich um die üblichen Oxalkylate handeln, wie sie durch Oxalkylierung, vorzugsweise Ethoxylierung und/oder Propoxylierung, von mindestens ein aktives H-Atom aufweisenden Verbindungen, vorzugsweise von ein- oder mehrwertigen Alkoholen, erhalten werden. Geeignete ein- und mehrwertige Alkohole sind die Alkanole (geradkettig oder verzweigt) mit vorzugsweise 4 bis 18 C-Atomen, Ethylenglykol, Propylenglykol, Diethylenglykol, Dipropylenglykol, Butandiol, Neopentylglykol, Glycerin, Trimethylolpropan, Erythrit, Pentaerythrit, Pentite und Hexite sowie die ($C_1$ bis $C_{12}$-alkyl)-Teilether der genannten mehrwertigen Alkohole. Die Alkanole und Glycerin, Ethylenglykol und Propylenglykol sowie deren ($C_1$ bis $C_{12}$-alkyl)-Teilether sind bevorzugt. Die bevorzugten Rohoxalkylate für das erfindungsgemäße Verfahren sind demnach solche, die aus den genannten ein- oder mehrwertigen Alkoholen durch Ethoxylierung mit 1 bis 30 mol, vorzugsweise 2 bis 20 mol, Ethylenoxid pro mol OH-Funktion im Alkohol, Propoxylierung mit 1 bis 30 mol, vorzugsweise 2 bis 20 mol, Propylenoxid pro mol OH-Funktion im Alkohol oder durch Ethoxylierung mit der genannten Ethylenoxidmenge und Propoxylierung mit der genannten Propylenoxidmenge hergestellt worden sind (unter Verwendung der in Rede stehenden Katalysatoren), wobei die genannten Ethoxylate besonders bevorzugt sind.

Die Erfindung wird nun an Beispielen noch näher erläutert.

Beispiel 1

Zur Bereitung eines $C_{18}$-Alkoholethoxylats mit 2 mol Ethylenoxid pro mol Alkohol mittels $SnCl_4$-Katalyse werden 560 g (2,0 mol) trockenes Octadecanol in einem Rührautoklaven vorgelegt und unter $N_2$-Atmosphäre bei 40 bis 60 °C mit 1,6 g $SnCl_4$ als Katalysator vermischt. Der Mischung werden bei 50 bis 80 °C 176 g Ethylenoxid (4,0 mol) im Laufe von 4 Stunden zudosiert. Die anfangs exotherme Reaktion wird durch einstündiges Nachrühren bei 80 °C beendet, was durch Konstanz des Druckes im Autoklav erkennbar ist. Das Rohoxalkylat hat einen Gehalt an Chlor (das im wesentlichen organisch gebundenes Chlor ist) von 0,25 Gew.-% und einen Sn-Gehalt von 0,20 Gew.-%.

Reinigung des Rohoxalkylats:

Zu 500 g Rohoxalkylat in einem Rührautoklaven werden unter $N_2$-Abdeckung 8,6 g Natriummethylat-Lösung (30gew.%ige Lösung von $CH_3ONa$ in Methanol) zugegeben und die Mischung 1 Stunde lang bei 130 °C gerührt. Die zugegebene Menge Natriummethylat entspricht der zweifach stöchiometrischen Menge, bezogen auf anwesendes Gesamtchlor. Nach Abkühlen der Mischung auf 70 °C werden 2,5 g Wasser zugesetzt, worauf die Mischung bei der angegebenen Temperatur mit 85gew.%iger wäßriger Phosphorsäure auf einen pH-Wert von 6,5 eingestellt wird. Die Mischung enthält eine Wassermenge von 0,6 Gew.-% (Gewichtsprozente bezogen auf das Gewicht der Mischung). Es folgt das Abdestillieren der flüchtigen Anteile (Wasser und Methanol) in der Mischung durch Anlegen von vermindertem Druck (Wasserstrahlvakuum) bei 90 °C über einen Zeitraum von 45 Minuten. Im letzten Arbeitsschritt wird die Mischung zur Abtrennung der grob-kristallin vorliegenden Salze in warmem Zustand filtriert. Das so gereinigte Ethylenoxid-Addukt bat einen Chlorgehalt von nur noch 0,0011 Gew.-%. Der Zinngehalt liegt bei 0,0003 Gew.-% (vergleiche nachstehende Tabelle, in der alle Beispiele bezüglich Verfahrensablauf und Ergebnis zusammengefaßt sind).

Beispiel 2

Zur Bereitung eines $C_{12/14}$-Alkoholethoxylats mit 8 mol Ethylenoxid pro mol Alkohol mittels $SbCl_5$-Katalyse werden 194 g (1,0 mol) trockener $C_{12/14}$-Alkohol in einem Rührautoklaven vorgelegt und unter $N_2$-Atmosphäre bei 40 bis 60 °C mit 0,9 g $SbCl_5$ als Katalysator vermischt. Die Bedingungen der Umsetzung mit 352 g Ethylenoxid (8,0 mol) und die Abreaktion des Ethylenoxids entsprechen denjenigen von Beispiel 1.

Reinigung des Rohoxalkylats:

Zu 500 g Rohoxalkylat in einem Rührautoklaven werden unter $N_2$-Abdeckung 11,0 g Natriumhydroxid-Lösung (25gew.%ig in Wasser) zugegeben und die Mischung 2 Stunden bei 120 °C gerührt. Die zugegebene Menge Natriumhydroxid entspricht der fünffach stöchiometrischen Menge, bezogen auf anwesendes Gesamtchlor. Nach Abkühlen der Mischung auf 90 °C wird mit 50gew.%iger wäßriger phosphoriger Säure auf einen pH-Wert von 4 eingestellt. Die Mischung enthält eine Wassermenge von 2,5 Gew.-%. Es folgt das Abdestillieren der flüchtigen Anteile (Wasser) und die Filtration der grob-kristallin vorliegenden Salze zur Gewinnung des Rein-Oxalkylats, wie in Beispiel 1 beschrieben.

Beispiel 3

Zur Bereitung eines Glycerin-Ethylenoxid-Propylenoxid-Mischoxalkylats mittels $SbCl_5$-Katalyse werden 92 g (1,0 mol) trockenes Glycerin in einem Rührautoklaven vorgelegt und unter $N_2$-Atmosphäre bei 40 bis 60 °C mit 1,3 g $SbCl_5$ als Katalysator vermischt. Der Mischung werden bei 60 bis 80 °C eine Mischung aus 264 g Ethylenoxid (6,0 mol) und 264 g Propylenoxid (4,5 mol) im Laufe von 5 Stunden zudosiert. Weitere Durchführung der Oxalkylierungsreaktion wie in Beispiel 1.

Reinigung des Rohoxalkylats:

Zu 500 g Rohoxalkylat in einem Rührautoklaven werden unter $N_2$-Abdeckung 11,9 g Natriummethylat-Lösung (30gew.%ig in Methanol) zugegeben und die Mischung 1 Stunde bei 140°C gerührt. Die zugegebene Menge Natriummethylat entspricht der vierfach stöchiometrischen Menge, bezogen auf anwesendes Gesamtchlor. Nach Abkühlen der Mischung auf 95 °C werden 5 g Wasser zugesetzt und mit $H_2SO_4$ (50gew.%ig in Wasser) auf pH 6,3 eingestellt. Die Mischung enthält eine Wassermenge von 1,5 Gew.-%. Entfernung der flüchtigen Anteile und Filtration wie in Beispiel 1.

Beispiel 4

Zur Bereitung eines n-Butanolethoxylats mit 15 mol Ethylenoxid pro mol Butanol mittels $SbCl_5$-Katalyse werden 74 g trockenes n-Butanol (1,0 mol) in Gegenwart von 2,4 g $SbCl_5$ unter den Bedingungen von Beispiel 1 mit 660 g Ethylenoxid (15,0 mol) umgesetzt.

Reinigung des Rohoxalkylats:

Zu 500 g Rohethoxylat in einem Rührautoklaven werden unter $N_2$-Abdeckung 27,3 g Kaliumhydroxid-Lösung (8,4gew.%ig in Wasser) sowie 5 g Ethanol zugegeben und die Mischung 2 Stunden bei 140 °C gerührt. Die zugegebene Menge Kaliumhydroxid entspricht der 1,5fach stöchiometrischen Menge, bezogen auf anwesendes Gesamtchlor. Nach geringem Abkühlen wird die Mischung mit in 10 g Wasser gelöster Oxalsäure auf pH 5 eingestellt. Die Mischung enthält eine Wassermenge von 7,0 Gew.-%. Entfernung der flüchtigen Anteile und Filtration wie in Beispiel 1.

Beispiel 5

Zur Bereitung eines $C_{12}$-Alkohol-Ethoxylats mit 4 mol Ethylenoxid pro mol Alkohol mittels $SbCl_5$-Katalyse werden 372 g (2,0 mol) trockenes Dodecanol in Gegenwart von 1,8 g $SbCl_5$ unter den Bedingungen von Beispiel 1 mit 352 g Ethylenoxid (8,0 mol) umgesetzt.

Reinigung des Rohoxalkylats:

Zu 500 g Rohoxalkylat in einem Rührautoklaven werden unter $N_2$-Abdeckung 8,4 g Kaliumhydroxid-Lösung (40gew.%ig in Wasser) zugegeben und die Mischung 2 Stunden bei 130 °C gerührt. Die zugegebene Menge Kaliumhydroxid entspricht der zweifach stöchiometrischen Menge, bezogen auf anwesendes Gesamtchlor. Nach Abkühlen der Mischung auf 50 °C wird mit 85gew.%iger wäßriger Phosphorsäure auf einen pH-Wert von 4,5 eingestellt. Die Mischung enthält eine Wassermenge von 2,8 Gew.-%. Entfernung der flüchtigen Anteile und Filtration wie in Beispiel 1.

Beispiel 6

Zur Bereitung eines $C_{12}$-Alkohol-Ethoxylats mit 8 mol Ethylenoxid pro mol Alkohol mittels $SnCl_4$-Katalyse werden 372 g (2,0 mol) trockenes Dodecanol in Gegenwart von 3,1 g $SnCl_4$ unter den Bedingungen von Beispiel 1 mit 704 g Ethylenoxid (16,0 mol) umgesetzt.

Reinigung des Rohoxalkylats:

Zu 500 g Rohoxalkylat in einem Rührautoklaven werden unter $N_2$-Abdeckung 7,1 g 25gew.%iger wäßriger Natriumhydroxid-Lösung zugegeben und die Mischung 4 Stunden bei 110 °C gerührt. Die zugegebene Menge Natriumhydroxid entspricht der zweifach stöchiometrischen Menge, bezogen auf anwesendes Gesamtchlor. Nach Abkühlen der Mischung auf 100 °C wird mit 85gew.%iger wäßriger Phosphorsäure auf einen pH-Wert von 6,5 eingestellt. Die Mischung enthält eine Wassermenge von 1,0 Gew.-%. Entfernung der flüchtigen Anteile und Filtration wie in Beispiel 1.

Vergleichsbeispiel A

Reinigung des Rohethoxylats von Beispiel 5:

Zu 200 g Rohethoxylat werden 1,0 g $Na_2HPO_4$ und 1,0 g Wasser zugegeben. Die Mischung wird 2 Stunden bei 120 °C gerührt, worauf sie zur Gewinnung des Ethoxylats filtriert wird.

Vergleichsbeispiel B

Reinigung des Rohethoxylats von Beispiel 6:

Zu 200 g Rohethoxylat werden 1,4 g $Na_2CO_3$ und 12 g Wasser zugegeben und die Mischung 4 Stunden bei 105 °C gerührt. Anschließend werden die flüchtigen Anteile unter vermindertem Druck und bei einer Temperatur bis zu 90 °C abdestilliert, worauf die Mischung zur Gewinnung des Ethoxylats filtriert wird.

In der nachfolgenden Tabelle sind, wie bereits erwähnt, alle Beispiele und die beiden Vergleichsbeispiele bezüglich Verfahrensablauf und erzieltem Reinigungseffekt zusammengefaßt.

**TABELLE**

| | Beispiel 1 | Beispiel 2 | Beispiel 3 | Beispiel 4 | Beispiel 5 | Beispiel 6 |
|---|---|---|---|---|---|---|
| Alkanol oder Polyol | $C_{18}$-Alkohol | $C_{12/14}$-Alkohol | Glycerin | Butanol | $C_{12}$-Alkohol | $C_{12}$-Alkohol |
| mol Epoxid/mol | 2 EO | 8 EO | 6 EO/4,5 PO | 15 EO | 4 EO | 8 EO |
| Katalysator | $SnCl_4$ | $SbCl_5$ | $SbCl_5$ | $SbCl_5$ | $SbCl_5$ | $SnCl_4$ |
| Gehalt an Chlor (Gew.-%) | 0,25 | 0,10 | 0,12 | 0,20 | 0,15 | 0,16 |
| Gehalt an Sb oder Sn (Gew.-%) | 0,20 Sn | 0,07 Sb | 0,08 Sb | 0,14 Sb | 0,10 Sb | 0,13 Sn |
| Alkali-Art | $CH_3ONa$ | NaOH | $CH_3ONa$ | KOH | KOH | NaOH |
| Stöchiometrischer Über- schuß, bezogen auf Chlor | 2fach | 5fach | 4fach | 1,5fach | 2fach | 2fach |
| Reaktionstemperatur/Zeit | 130 °C/1 h | 120 °C/2 h | 140 °C/1 h | 140 °C/2 h | 130 °C/2 h | 110 °C/4 h |
| Säure | $H_3PO_4$ | $H_3PO_3$ | $H_2SO_4$ | Oxalsäure | $H_3PO_4$ | $H_3PO_4$ |
| pH-Wert | 6,5 | 4 | 6,3 | 5 | 4,5 | 6,5 |
| Wassergehalt der Mischung (Gew.-%) | 0,6 | 2,5 | 1,5 | 7,0 | 2,8 | 1,0 |
| Ergebnis nach der Reinigung: | | | | | | |
| Gehalt an Chlor (Gew.-%) | 0,0011 | 0,0012 | 0,0020 | 0,0010 | 0,0015 | 0,0008 |
| Gehalt an Sb oder Sn (Gew.-%) | 0,0003 | <0,0001 | 0,0008 | <0,0001 | 0,0002 | 0,0002 |

(Fortsetzung)

EP 0 394 984 B1

EP 0 394 984 B1

TABELLE (Fortsetzung)

| | Vergleichsbeispiel A | Vergleichsbeispiel B |
|---|---|---|
| Alkanol oder Polyol | $C_{12}$-Alkohol | $C_{12}$-Alkohol |
| mol Epoxid/mol | 4 EO | 8 EO |
| Katalysator | $SbCl_5$ | $SnCl_4$ |
| Gehalt an Chlor (Gew.-%) | 0,15 | 0,16 |
| Gehalt an Sb oder Sn (Gew.-%) | 0,10 Sb | 0,13 Sn |
| Alkali-Art | $Na_2HPO_4$ | $Na_2CO_3$ |
| Stöchiometrischer Überschuß, bezogen auf Chlor | --- | 1,5fach |
| Reaktionstemperatur/Zeit | 120 °C/2 h | 105 °C/4 h |
| Säure | --- | --- |
| pH-Wert | 5,9 | 9,0 |
| Wassergehalt der Mischung (Gew.-%) | 0,5 | 6,0 |
| Ergebnis nach der Reinigung: | | |
| Gehalt an Chlor (Gew.-%) | 0,1500 | 0,1200 |
| Gehalt an Sb oder Sn (Gew.-%) | 0,0130 | 0,0002 |

Wie die Beispiele und Vergleichsbeispiele zeigen, werden mit der erfindungsgemäßen Arbeitsweise unerwartet reine Produkte sowohl bezüglich Halogen- als auch bezüglich Metallgehalt erhalten.

## Patentansprüche

1. Verfahren zur Reinigung eines Alkylenoxid-Adduktes, das unter Verwendung von halogenhaltigen Metallkatalysatoren hergestellt worden ist und Halogen enthaltende Verbindungen sowie Metalle als Verunreinigung enthält, dadurch gekennzeichnet, daß man

a) das zu reinigende Alkylenoxid-Addukt mit Alkalimetallverbindungen aus der Gruppe der Oxide, Hydroxide und Alkoholate in einer mindestens 1,05fachen stöchiometrischen Menge, bezogen auf die im Addukt vorliegende Halogenmenge, bei erhöhter Temperatur in Kontakt bringt,

b) das alkalische Produkt vom Schritt a) mit einer Säure auf einen pH-Wert von höchstens 7 bringt, mit der Maßgabe, daß im nun vorliegenden Produkt eine Wassermenge von mindestens 0,1 Gew.-%, bezogen auf das Gewicht des Produktes, anwesend ist,

c) aus dem nach den Schritten a) und b) erhaltenen Produkt das Wasser und andere Lösungsmittel für die in den Schritten a) und b) gebildeten und im Alkylenoxid-Addukt unlöslichen Salze entfernt, und man

d) aus dem im Schritt c) erhaltenen Produkt, das im wesentlichen aus Alkylenoxid-Addukt und den genannten Salzen besteht, durch Abtrennung dieser Salze das angestrebte reine Alkylenoxid-Addukt gewinnt.

2. Verfahren nach Anspruch 1, dadurch gekennzeichnet, daß man im Schritt a) ein Alkalimetallhydroxid oder ein Alkalimetall-$C_1$ bis $C_4$-alkoholat einsetzt.

8

**3.** Verfahren nach Anspruch 1 oder 2, dadurch gekennzeichnet, daß man im Schritt a) die Alkalimetallverbindung in einer 1,3- bis 5fachen stöchiometrischen Menge einsetzt.

**4.** Verfahren nach einem oder mehreren der Ansprüche 1 bis 3, dadurch gekennzeichnet, daß man im Schritt a) die Alkalimetallverbindungen in Form einer wäßrigen, $C_1$ bis $C_4$-alkoholischen oder in einer $C_1$ bis $C_4$-alkoholisch/wäßrigen Lösung einsetzt.

**5.** Verfahren nach einem oder mehreren der Ansprüche 1 bis 4, dadurch gekennzeichnet, daß man im Schritt b) auf einen pH-Wert von höchstens 7 einstellt, mit der Maßgabe, daß das nun vorliegende Produkt eine Wassermenge von 0,5 bis 10 Gew.-% enthält.

**6.** Verfahren nach einem oder mehreren der Ansprüche 1 bis 5, dadurch gekennzeichnet, daß man im Schritt b) den pH-Wert mit einer Säure aus der Gruppe der Phosphorsäuren, Schwefelsäuren, Oxalsäure und Zitronensäure einstellt.

**7.** Verfahren nach einem oder mehreren der Ansprüche 1 bis 6, dadurch gekennzeichnet, daß man im Schritt a) das Inkontaktbringen des zu reinigenden Alkylenoxid-Adduktes mit der Alkalimetallverbindung bei einer Temperatur von 80 bis 180 °C und 0,5 bis 5 Stunden lang durchführt und im Schritt b) das Einstellen des pH-Wertes mit der Säure bei einer Temperatur von 20 bis 150 °C durchführt.

**8.** Verfahren nach Anspruch 1 dadurch gekennzeichnet, daß man
a) das zu reinigende Alkylenoxid-Addukt mit einem Alkalimetallhydroxid oder einem Alkalimetall-$C_1$ bis $C_4$-alkoholat in einer 1,3- bis 5fachen stöchiometrischen Menge in Form einer wäßrigen, $C_1$ bis $C_4$-alkoholischen oder in einer $C_1$ bis $C_4$-alkoholisch/wäßrigen Lösung versetzt und die Mischung bei einer Temperatur von 80 bis 180 °C 0,5 bis 5 Stunden lang rührt,
b) das alkalische Produkt vom Schritt a) auf eine Temperatur von 20 bis 150 °C bringt und bei dieser Temperatur mit einer Säure aus der Gruppe der Phosphorsäuren, Schwefelsäuren, Oxalsäure und Zitronensäure auf einen pH-Wert von 3 bis 7 einstellt, mit der Maßgabe, daß im nun vorliegenden Produkt eine Wassermenge von mindestens 0,1 Gew.-% anwesend ist,
c) aus dem nach den Schritten a) und b) erhaltenen Produkt die Lösungsmittel Wasser und $C_1$ bis $C_4$-Alkohol destillativ entfernt, und man
d) das im Schritt c) erhaltene Produkt filtriert oder zentrifugiert.

**9.** Verfahren nach Anspruch 1, dadurch gekennzeichnet, daß man
a) das zu reinigende Alkylenoxid-Addukt mit einem Alkalimetallhydroxid oder einem Alkalimetall-$C_1$ bis $C_4$-alkoholat in einer 1,3- bis 5fachen stöchiometrischen Menge in Form einer wäßrigen, $C_1$ bis $C_4$-alkoholischen oder in einer $C_1$ bis $C_4$-alkoholisch/wäßrigen Lösung versetzt und die Mischung bei einer Temperatur von 100 bis 150 °C 0,5 bis 5 Stunden lang rührt,
b) das alkalische Produkt vom Schritt a) auf eine Temperatur von 50 bis 130 °C bringt und bei dieser Temperatur mit einer Säure aus der Gruppe der Phosphorsäuren, Schwefelsäuren und Oxalsäure auf einen pH-Wert von 3 bis 7 einstellt, mit der Maßgabe, daß im nun vorliegenden Produkt eine Wassermenge von 0,5 bis 10 Gew.-% anwesend ist,
c) aus dem nach den Schritten a) und b) erhaltenen Produkt die Lösungsmittel Wasser und $C_1$ bis $C_4$-Alkohol destillativ entfernt, und man
d) das im Schritt c) erhaltene Produkt filtriert oder zentrifugiert.

## Claims

**1.** A process for the purification of an alkylene oxide adduct which has been prepared using halogen-containing metal catalysts and contains halogen-containing compounds and also metals as impurities, which comprises
a) bringing the alkylene oxide adduct which is to be purified into contact at elevated temperature with at least 1.05 times the stoichiometric amount, relative to the amount of halogen present in the adduct, of an alkali metal compound from the group consisting of oxides, hydroxides and alcoholates,
b) adjusting the pH of the alkaline product from step a) to a maximum of 7 with an acid, with the proviso that the product then obtained contains at least 0.1 % by weight of water, relative to the weight of product,
c) freeing the product obtained after steps a) and b) from water and other solvents for the salts which were formed in steps a) and b) and are insoluble in the alkylene oxide adduct, and

d) isolating the desired pure alkylene oxide adduct from the product obtained in step c), this product comprising essentially alkylene oxide adduct and the abovementioned salts, by separating off the said salts.

2. The process as claimed in claim 1, wherein step a) employs an alkali metal hydroxide or an alkali metal $C_1$ to $C_4$-alcoholate.

3. The process as claimed in claim 1 or 2, wherein step a) employs the alkali metal compound in 1.3 to 5 times the stoichiometric amount.

4. The process as claimed in one or more of claims 1 to 3, wherein step a) employs the alkali metal compounds in the form of an aqueous, $C_1$ to $C_4$-alcoholic or $C_1$ to $C_4$-alcoholic/aqueous solution.

5. The process as claimed in one or more of claims 1 to 4, wherein the pH in step b) is adjusted to at most 7, with the proviso that the product which is then present has a water content of 0.5 to 10 % by weight.

6. The process as claimed in one or more of claims 1 to 5, wherein the pH in step b) is adjusted using an acid from the group consisting of phosphorus acids, sulfur acids, oxalic acid and citric acid.

7. The process as claimed in one or more of claims 1 to 6, wherein step a) brings the alkylene oxide adduct which is to be purified into contact with the alkali metal compound at a temperature of 80 to 180°C and for 0.5 to 5 hours and step b) adjusts the pH, using the acid, at a temperature of 20 to 150°C.

8. The process as claimed in claim 1, wherein

a) an alkali metal hydroxide or an alkali metal $C_1$ to $C_4$-alcoholate is added in an amount of 1.3 to 5 times the stoichiometric amount in the form of an aqueous, $C_1$ to $C_4$-alcoholic or $C_1$ to $C_4$-alcoholic/aqueous solution to the alkylene oxide adduct which is to be purified and the mixture is stirred for 0.5 to 5 hours at a temperature of 80 to 180°C,

b) the alkaline product from step a) is brought to a temperature of 20 to 150°C and its pH is adjusted to 3 to 7 at this temperature using an acid from the group consisting of phosphorus acids, sulfur acids, oxalic acid and citric acid, with the proviso that the water content of the product which is then present is at least 0.1 % by weight,

c) the product obtained from steps a) and b) is freed from the solvents water and $C_1$ to $C_4$-alcohol by distillation, and

d) the product obtained in step c) is filtered or centrifuged.

9. The process as claimed in claim 1, wherein

a) an alkali metal hydroxide or an alkali metal $C_1$ to $C_4$-alcoholate is added in an amount of 1.3 to 5 times the stoichiometric amount in the form of an aqueous, $C_1$ to $C_4$-alcoholic or $C_1$ to $C_4$-alcoholic/aqueous solution to the alkylene oxide adduct which is to be purified and the mixture is stirred for 0.5 to 5 hours at a temperature of 100 to 150°C,

b) the alkaline product from step a) is brought to a temperature of 50 to 130°C and its pH is adjusted to 3 to 7 at this temperature using an acid from the group consisting of phosphorus acids, sulfur acids and oxalic acid, with the proviso that the water content of the product which is then present is 0.5 to 10 % by weight,

c) the product obtained from steps a) and b) is freed from the solvents water and $C_1$ to $C_4$-alcohol by distillation, and

d) the product obtained in step c) is filtered or centrifuged.

**Revendications**

1. Procédé pour purifier un produit d'addition d'un oxyde d'alkylène, qui a été produit avec utilisation de catalyseurs à base de métaux, contenant de l'halogène, et qui contient comme impureté des composés contenant de l'halogène ainsi que des métaux , procédé caractérisé en ce que

(a) on met le produit d'addition d'oxyde d'alkylène, à purifier, en contact à température élevée avec des composés de métaux alcalins choisis dans l'ensemble formé par des oxydes des hydroxydes et des alcoolates, en une quantité d'au moins 1,05 fois la quantité stoéchiométrique, par rapport à la quantité d'halogène présent dans le produit d'addition;

(b) on amène à l'aide d'un acide le produit alcalin de l'étape (a) à une valeur de pH de 7 au maximum, à la condition qu'il y ait dans le produit alors présent une quantité d'eau égale au minimum 0,1 % en poids, par rapport au poids du produit,

(c) on enlève du produit, obtenu selon les étapes (a) et (b) , l'eau et d'autres solvants des sels ,formés au cours des étapes (a) et (b) et insolubles dans le produit d'addition d'oxyde d'alkylène, et,

(d) à partir du produit obtenu dans l'étape (c) et qui consiste essentiellement en un produit d'addition d'un oxyde d'alkylène et en les sels précités, on obtient,en séparant ces sels, le produit pur voulu d'addition d'oxyde d'alkylène.

2. Procédé selon la revendication 1 , caractérisé en ce qu'on utilise dans l'étape (a) un hydroxyde de métal alcalin ou un alcoolate (en $C_1$ à $C_4$) de métal alcalin .

3. Procédé selon la revendication 1 ou 2 , caractérisé en ce qu'on utilise dans l'étape (a) le composé de métal alcalin en une quantité représentant 1,3 à 5 fois la quantité stoéchiométrique .

4. Procédé selon une ou plusieurs des revendications 1 à 3 ,caractérisé en ce qu'on utilise dans l'étape (a) les composés de métaux alcalins sous forme d'une solution aqueuse, dans de l'alcool en $C_1$ à $C_4$ ou dans une solution hydro-alcoolique en $C_1$ à $C_4$ .

5. Procédé selon une ou plusieurs des revendications 1 à 4 , caractérisé en ce qu'on ajuste dans l'étape (b) à une valeur de pH de 7 au maximum , à la condition que le produit alors obtenu contienne une quantité d'eau de 0,5 à 10 % en poids .

6. Procédé selon une ou plusieurs des revendications 1 à 5 , caractérisé en ce que,dans l'étape (b), on ajuste la valeur du pH à l'aide d'un acide choisi parmi l'ensemble formé par les acides phosphoriques , les acides sulfuriques, l'acide oxalique et l'acide citrique .

7. Procédé selon une ou plusieurs des revendications 1 à 6 , caractérisé en ce qu'on conduit dans l'étape (a) la mise en contact du produit d'addition d'un oxyde d'alkylène , à purifier,avec le composé de métal alcalin à une température de 80 à 180 °C durant 0,5 à 5 heures et en ce qu'on effectue dans l'étape (b) l'ajustement du pH avec l'acide en opérant à une température de 20 à 150 °C.

8. Procédé selon la revendication 1 , caractérisé en ce que :

(a)au produit d'addition d'un oxyde d'alkylène,à purifier, on ajoute un hydroxyde de métal alcalin ou un alcoolate ( en $C_1$ à $C_4$) de métal alcalin en une quantité représentant 1,3 à 5 fois la quantité stoéchiométrique,sous forme d'une solution aqueuse, dans un alcool en $C_1$ à $C_4$ ou en solution hydro-alcoolique en $C_1$ à $C_4$) et l'on agite le mélange duarnt 0,5 à 5 heures à une température de 80 à 180 °C;

(b) on porte le produit alcalin de l'étape (a) à une température de 20 à 150 °C et l'on ajuste à cette température à une valeur de pH de 3 à 7 à l'aide d'un acide choisui parmi les acides phosphoriques ,les acides sulfuriques, l'acide oxalique et l'acide citrique,à la condition qu'il y ait dans le produit alors présent une quantité d'eau d'au moins 0,1 % en poids ;

(c) du produit obtenu selon les étapes (a) et (b) ,on enlève par distillation les solvants,l'eau et l'alcool en $C_1$ 5 à $C_4$, et

(d) on filtre ou centrifuge le produit obtenu dans l'étape (c) .

9. Procédé selon la revendication 1 ,caractérisé en ce que:

(a) au produit d'addition d'un oxyde d'alkylène,à purifier,on ajoute un hydroxyde de métal alcalin ou un alcoolate (en $C_1$ à $C_4$) de métal alcalin, en une quantité représentant 1,3 à 5 fois la quantité stoéchiométrique,sous forme d'une solution aqueuse , dasn un alcool en $C_1$à $C_4$ ou en solution hydro-alcoolique en $C_1$ à $C_4$, et l'on agite le mélange durant 0,5 à 5 heures à une température de 100 à 150 °C ;

(b) on porte le produit alcalin de l'étape (a) à une température de 50 à 130 °C et,à cette température,on ajuste à une valeur de pH de 3 à 7 à l'aide d'un acide choisi parmi les acides phosphoriques, les acides sulfuriques et l'acide oxalique, à la condition que,dans le produit alors obtenu,il y ait présence d'une quantité d'eau de 0,5 à 10 % en poids ;

(c) on enlève par distillation,du produit obtenu selon les étapes (a) et (b) ,les solvants que sont l'eau et un alcool en $C_1$ à $C_4$ ;et

(d) on filtre ou centrifuge le produit obtenu dans l'étape (c) .